# EUROPEAN PATENT SPECIFICATION

(11) **EP 4 160 362 B1**
(45) Date of publication and mention of the grant of the patent: **11.12.2024**
(21) Application number: 22198592.2
(22) Date of filing: 29.09.2022
(51) Int. Cl.: G06F 3/01, G06V 40/18, G02B 27/00, G06V 10/94, G06V 40/19

(54) **GAZE DEFECT COMPENSATION**
BLICKDEFEKTKOMPENSATION
COMPENSATION DE DÉFAUT DE REGARD

(30) Priority: 30.09.2021 SE 2151198
(43) Date of publication of application: 05.04.2023
(73) Proprietor: Tobii AB, 182 17 Danderyd (SE)
(72) Inventor: Johansson Tornéus, Daniel, 18217 Danderyd (SE)
(74) Representative: Novagraaf Group

(56) References cited:
- US-A1- 2017 123 526
- US-A1- 2020 150 759
- US-A1- 2020 258 295
- ARTAL PABLO: "Optics of the eye and its impact in vision: a tutorial", ADVANCES IN OPTICS AND PHOTONICS, vol. 6, no. 3, 24 September 2014 (2014-09-24), pages 340, XP093021674, Retrieved from the Internet <URL:https://www.researchgate.net/profile/Pablo-Artal/publication/265971301_Optics_of_the_eye_and_its_impact_in_vision_A_tutorial/links/54256a7e0cf2e4ce94037f55/Optics-of-the-eye-and-its-impact-in-vision-A-tutorial.pdf> [retrieved on 20230207], DOI: 10.1364/AOP.6.000340
- PASCOLO P ET AL: "Dynamical models of the human eye and strabismus", CHAOS SOLUTIONS AND FRACTALS, PERGAMON, OXFORD, GB, vol. 41, no. 5, 15 September 2009 (2009-09-15), pages 2463 - 2470, XP026237913, ISSN: 0960-0779, [retrieved on 20081101], DOI: 10.1016/J.CHAOS.2008.09.032

## Description

### Field

The present disclosure generally relates to the field of eye tracking. In particular, the present disclosure relates to eye tracking systems and methods for providing accurate eye tracking in the presence of health-related eye defects.

### Background

In eye tracking applications, digital images are retrieved of the eyes of a user and the digital images are analysed in order to estimate the gaze direction of the user. The estimation of the gaze direction may be based on computer-based image analysis of features of the imaged eye. One known example method of eye tracking includes the use of infrared light and an image sensor. The infrared light is directed towards the pupil of a user and the reflection of the light is captured by an image sensor.

Many eye tracking systems estimate gaze direction based on identification of a pupil position together with glints or corneal reflections. Eye tracking systems may include a calibration sequence for defining an eye tracking model that can map the pupil position and glints to gaze direction. However, such eye tracking models can suffer from poor performance for users with health-related eye defects.

Portable or wearable eye tracking devices have also been previously described. One such eye tracking system is described in U.S. Pat. No. 9,041,787. A wearable eye tracking device is described using illuminators and image sensors for determining gaze direction.

US 2020/150759 A1 describes a line-of-sight detection apparatus including a first direction determining section that determines a first direction that is a direction of a line of sight of a first eye of a subject; a second direction determining section that determines a second direction that is a direction of a line of sight of a second eye of the subject; an assuming section that assumes a gaze direction of the subject based on at least one of (i) a manipulation by the subject of a device provided with a line-of-sight measurement apparatus that measures a line of sight of the subject, (ii) a state of the device, and (iii) an orientation of a face of the subject; and a first judging section that compares the assumed gaze direction, the first direction, and the second direction, and judges which of the first eye and the second eye is a dominant eye.

ARTAL PABLO: "Optics of the eye and its impact in vision: a tutorial", ADVANCES IN OPTICS AND PHOTONICS, vol. 6, no. 3, 24 September 2014 (2014-09-24), page 340, XP093021674, DOI: 10.1364/AOP.6.000340 describes the main characteristics of the eye's geometry and optics, as well as the impact on vision under a variety of normal conditions. PASCOLO PET AL: "Dynamical models of the human eye and strabismus", CHAOS SOLUTIONS AND FRACTALS, PERGAMON, OXFORD, GB, vol. 41, no. 5, 15 September 2009 (2009-09-15), pages 2463-2470, XP026237913, ISSN: 0960-0779, DOI: 10.1016/ J.CHAOS. 2008.09.032 describes pathological dynamics by using a dynamical model of the human eye to the case of strabismus.

US 2017/0123526 A1 discloses a system and method for tracking a user's eye using structured light. The structured light system is calibrated by training a model of surface of the user's eye. A structured light emitter projects a structured light pattern ( e.g., infrared structured light) onto a portion of the surface of the eye. From the viewpoint of a camera, the illumination pattern appears distorted. Based on the distortion of the illumination pattern in the captured image, the eye tracking system can determine the shape of the portion of the user's eye that the structured light is incident upon. By comparing the determined shape of the portion of the user's eye to the model, the orientation of the eye may be determined.

### Summary

According to a first aspect of the present disclosure there is provided an eye tracking system according to claim 1.

The eye tracking system can advantageously implement a gaze dependent eye-model that applies a different calculation process depending on a direction of the optical axis / user's gaze. By selecting one of a plurality of eye models based on a direction of the optical axis, the eye tracking system can advantageously compensate for Strabismus or other gaze related defects for one or both eyes.

The controller may be configured to select one of a plurality of predetermined eye models based on the direction of the optical axis.

The controller may be configured to select one of a plurality of eye models by: selecting a gaze offset value from a plurality of gaze offset values based on the direction of the optical axis; and applying the selected gaze offset value to a baseline eye model.

The controller may be configured to select one of a plurality of eye models by: selecting a gaze offset value from a plurality of gaze offset values based on the direction of the optical axis; and applying the selected gaze offset value to the selected predetermined eye model.

The controller may be configured to determine the plurality of eye models by determining, during a calibration process: a plurality of eye modelling parameter sets defining a corresponding plurality of predetermined eye models for a corresponding plurality of stimulus points or regions of stimulus points; a baseline eye model and a plurality of gaze offset values for a corresponding plurality of stimulus points or regions of stimulus points, wherein the gaze offset values are for applying to the baseline eye model; or a plurality of eye modelling parameter sets defining a corresponding plurality of predetermined eye models for a corresponding plurality of regions of stimulus points, and a plurality of gaze offset values for corresponding subsets of the regions of stimulus points, wherein the gaze offset values are for applying to one of the predetermined eye models.

The controller may be configured to: cause a plurality of stimulus points to be displayed one at a time to a user; and receive eye measurement data for each stimulus point.

The plurality of stimulus points may comprise 5 or more stimulus points.

Each of: the plurality of eye modelling parameter sets; and / or the plurality of gaze offset values, may correspond to: each stimulus point; or a region of stimulus points.

The controller may be configured to: determine a gaze vector for the left eye of the user; determine a gaze vector for the right eye of the user; determine a weighting for each of the left eye gaze vector and the right eye gaze vector based on the selected eye model for the respective left and right eye; and apply the weighting to the gaze vector of each of the left eye and the right eye to provide a combined gaze vector.

The controller may be configured to determine the weighting based on a magnitude of the gaze offset value associated with the selected eye model.

The controller may be configured to determine the weighting as dependent on a variation of the gaze offset values associated with the selected eye model relative to neighbouring values of the plurality of gaze offset values.

The controller may be configured to determine the weighting as dependent on a variation of the values of the eye modelling parameter sets associated with the selected eye model relative to neighbouring values of the plurality of eye modelling parameter sets.

The controller may be configured to determine a plurality of weightings during the calibration process, each weighting corresponding to each of: the plurality of eye modelling parameter sets; the plurality of gaze offset values; and / or the plurality of stimulus points. Each weighting may comprise a value from 0 to 1.

The controller may be further configured to: cause the plurality of stimulus points to be re-displayed one at a time to the user; and for each stimulus point that is displayed: receive eye measurement data; select the eye model corresponding to the stimulus point; calculate a gaze vector using the selected eye model and the eye measurement data; calculate a difference between the calculated gaze vector and the known gaze vector corresponding to the stimulus point; and determine a weighting based on the difference.

According to a second aspect of the present disclosure there is provided a head-mounted device comprising any eye tracking system as disclosed herein.

According to a third aspect of the present disclosure, there is provided a method of eye tracking according to claim 13.

According to a fourth aspect of the present disclosure, there is provided a method of calibrating an eye tracking system according to claim 14.

According to a fifth aspect of the present disclosure there is provided one or more non-transitory computer-readable storage media storing computer-executable instructions that, when executed by a computing system, causes the computing system to perform any method disclosed herein.

There may be provided a computer program, which when run on a computer, causes the computer to configure any apparatus, including a circuit, controller or device disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

The computer program may be provided on a computer readable medium, which may be a physical computer readable medium such as a disc or a memory device, or may be embodied as a transient signal. Such a transient signal may be a network download, including an internet download. There may be provided one or more non-transitory computer-readable storage media storing computer-executable instructions that, when executed by a computing system, causes the computing system to perform any method disclosed herein.

### Brief Description of the Drawings

One or more embodiments will now be described by way of example only with reference to the accompanying drawings in which:
Figure 1 shows a schematic view of an eye tracking system which may be used to capture a sequence of images that can be used by example embodiments of the present disclosure;
Figure 2 shows an example image of a pair of eyes;
Figure 3 illustrates schematically a difference between an optical axis and a gaze vector for an eye; and
Figure 4 illustrates measured gaze directions for various stimuli for a left eye of a user suffering from severe Strabismus;
Figure 5 illustrates an eye tracking system according to an embodiment of the present disclosure;
Figures 6A to 6C illustrate a calibration routine for determining a plurality of eye models;
Figure 7 illustrates schematically a method according to an embodiment of the present disclosure; and
Figure 8 illustrates schematically a method of calibrating an eye tracking system according to an embodiment of the present disclosure.

### Detailed Description

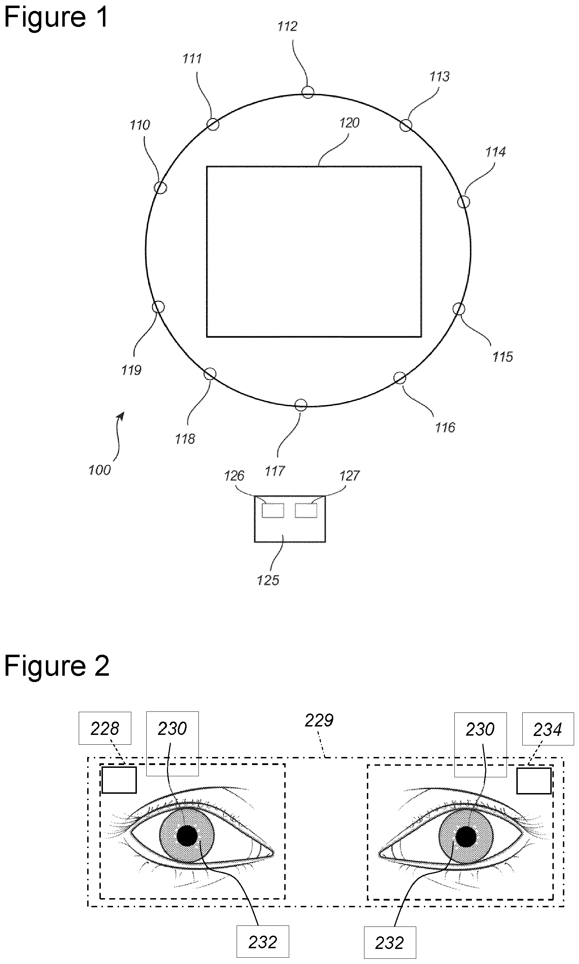

Figure 1 shows a simplified view of an eye tracking system 100 (which may also be referred to as a gaze tracking system) in a head-mounted device in the form of a virtual or augmented reality (VR or AR) device or VR or AR glasses or anything related, such as extended reality (XR) or mixed reality (MR) headsets. The system 100 comprises an image sensor 120 (e.g. a camera) for capturing images of the eyes of the user. The system may optionally include one or more illuminators 110-119 for illuminating the eyes of a user, which may for example be light emitting diodes emitting light in the infrared frequency band, or in the near infrared frequency band and which may be physically arranged in a variety of configurations. The image sensor 120 may for example be an image sensor of any type, such as a complementary metal oxide semiconductor (CMOS) image sensor or a charged coupled device (CCD) image sensor. The image sensor may consist of an integrated circuit containing an array of pixel sensors, each pixel containing a photodetector and an active amplifier. The image sensor may be capable of converting light into digital signals. In one or more examples, it could be an Infrared image sensor or IR image sensor, an RGB sensor, an RGBW sensor or an RGB or RGBW sensor with IR filter.

The eye tracking system 100 may comprise circuitry or one or more controllers 125, for example including a receiver 126 and processing circuitry 127, for receiving and processing the images captured by the image sensor 120. The circuitry 125 may for example be connected to the image sensor 120 and the optional one or more illuminators 110-119 via a wired or a wireless connection and be co-located with the image sensor 120 and the one or more illuminators 110-119 or located at a distance, e.g. in a different device. In another example, the circuitry 125 may be provided in one or more stacked layers below the light sensitive surface of the light sensor 120.

The eye tracking system 100 may include a display (not shown) for presenting information and/or visual stimuli to the user. The display may comprise a VR display which presents imagery and substantially blocks the user's view of the real-world or an AR display which presents imagery that is to be perceived as overlaid over the user's view of the real-world.

The location of the image sensor 120 for one eye in such a system 100 is generally away from the line of sight for the user in order not to obscure the display for that eye. This configuration may be, for example, enabled by means of so-called hot mirrors which reflect a portion of the light and allows the rest of the light to pass, e.g. infrared light is reflected, and visible light is allowed to pass.

While in the above example the images of the user's eye are captured by a head-mounted image sensor 120, in other examples the images may be captured by an image sensor that is not head-mounted. Such a non-head-mounted system may be referred to as a remote system.

In an eye tracking system, a gaze signal can be computed for each eye of the user (left and right). The quality of these gaze signals can be reduced by disturbances in the input images (such as image noise) and by incorrect algorithm behaviour (such as incorrect predictions). A goal of the eye tracking system is to deliver a gaze signal that is as good as possible, both in terms of accuracy (bias error) and precision (variance error). For many applications it can be sufficient to deliver only one gaze signal per time instance, rather than both the gaze of the left and right eyes individually. Further, the combined gaze signal can be provided in combination with the left and right signals. Such a gaze signal can be referred to as a combined gaze signal.

Figure 2 shows a simplified example of an image 229 of a pair of eyes, captured by an eye tracking system such as the system of Figure 1. The image 229 can be considered as including a right-eye-image 228, of a person's right eye, and a left-eye-image 234, of the person's left eye. In this example the right-eye-image 228 and the left-eye-image 234 are both parts of a larger image of both of the person's eyes. In other examples, separate image sensors may be used to acquire the right-eye-image 228 and the left-eye-image 234.

The system may employ image processing (such as digital image processing) for extracting features in the image. The system may for example identify a position of the pupil 230 in the one or more images captured by the image sensor. The system may determine the position of the pupil 230 using a pupil detection process. The system may also identify corneal reflections 232 located in close proximity to the pupil 230. The system may estimate a corneal centre or eye ball centre based on the corneal reflections 232. For example, the system may match each of the individual corneal reflections 232 for each eye with a corresponding illuminator and determine the corneal centre of each eye based on the matching.

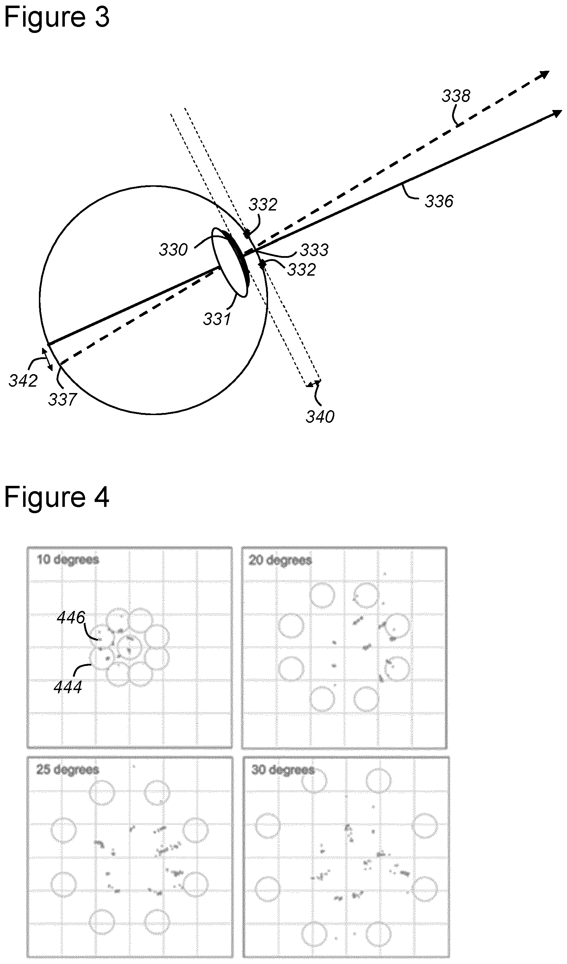

Figure 3 illustrates an example calculation of a gaze vector 338 for a user's eye based on the corneal reflections 332 and the position of the pupil 330 as determined by the image processing described in relation to Figure 2. To a first approximation, the eye tracking system may determine an optical axis 336 of the eye of the user as the vector passing through a centre of the pupil 330 and the corneal centre 333. However, the optical axis 336 does not typically align with the gaze vector 338 due to imperfections in the imaging system and / or anatomical variations in the shape and geometry of the human eye. The gaze vector 338 passes from the fovea 337 of the eye trough the centre of the pupil 330. The difference between the gaze vector 338 and the optical axis can depend on a pupil plane offset (PPO) 340 defining the distance of the plane of the pupil to the tangential plane at the front surface of the cornea /eye (in other words the depth of the pupil in the eye). The PPO can vary depending on the user and may not be directly measurable by the eye tracking system. The PPO 340 may act as a scalar multiplier component between the optical axis 336 and the gaze vector 338. In addition, as illustrated, a position of the fovea 337 may not lie on the optical axis and instead be defined by a fovea offset (FO) 342 from the optical axis 336. The FO 342 may comprise a vertical component (FO_{y}) and horizontal component (FOₓ), however we will simply refer to it herein as FO 342. The FO may act as a scalar offset component between the optical axis 336 and the gaze vector 338.

The eye tracking system may perform a calibration routine to estimate the PPO and FO for each individual user, prior to eye tracking. During calibration, one or more stimulus points (for example 5) at known positions / gaze angles may be displayed in turn on a screen and the user may be instructed to direct their gaze to each point as it is displayed. The eye tracking system can then compare the determined optical axis 336 to the true known gaze direction 338 (assuming that the user is looking at the correct stimulus point) to determine the PPO 340 and the FO 342. In this way, the eye tracking system can determine a personalised eye gaze tracking model (referred to herein as eye model) for each user based on the determined constants PPO 340 and FO 342.

The system can then determine a gaze vector (which may also be referred to as a gaze ray) for each eye. In some examples, the gaze vector may be based on a gaze origin and gaze direction which can be determined from the respective glint to illuminator matching / corneal centres, the determined pupil position and the calibrated constants (as defined by the eye tracking model). The gaze vector for each eye may be combined to provide a combined gaze vector.

The above-described process for determining the gaze vector is an example of a pupil centre corneal reflection (PCCR) approach. It will be appreciated that variations and alternatives to the above approach for determining an eye model for images of a user's eye are known in the art and that the present disclosure is not limited to any specific approach.

The above approach for determining a single calibrated eye model for each user can suffer performance issues when a user has a health-related eye defect. One such eye defect is Strabismus or "Lazy-Eye". 4% of the population suffers from Strabismus, which is characterized by an inability to always rotate both eyes towards a point of focus. The effect can be varied across individuals, which creates an additional layer of difficulty for eye-tracking. The effect can appear as: improper movement of one or both eyes in one or more directions; limited range of movement of one or both eyes; scaled movement relative to point of focus for one or both eyes; a constant offset in gaze for one eye; different constant offsets in both eyes depending on if the user is looking to the left or right; both eyes being cross-eyed (converged too close), non-constant offsets and / or many other effects. In some examples, Strabismus is non-uniform with effects varying greatly across the range of motion of the eye. In some examples Strabismus is inconsistent, in that the same stimulus point may not necessarily result in the same eye movement.

Figure 4 illustrates measured gaze directions for various stimuli for a left eye of a user suffering from severe Strabismus. Each quadrant shows the stimuli as a ring of circles 444 corresponding to a fixed angle deviation from the eye centre in 8 different directions. The angular deviation increases for each quadrant, as labelled in the figure. The measured gaze directions are indicated as points 446. For the 10-degree stimulus (top-left quadrant), the user's left eye exhibits a left-bias by only moving in a small area to the left of centre. However, for the 20-degree stimulus (top-right quadrant), a larger movement is recorded but with a right-bias to the right of centre. In general, the left eye is moving independently of where the user is expected to look. Furthermore, the left eye doesn't move beyond a range of ~ 15 degrees.

As a result of the varied nature of Strabismus, the personalised calibrated eye models described above suffer from performance issues for users exhibiting Strabismus. One approach for addressing Strabismus is for a user to disable the tracking for an affected eye. However, this approach severely limits the performance for unaffected gaze directions as combined gaze performance is better than single eye performance in most cases. This approach also fails to compensate for user's with Strabismus in both eyes.

The systems and methods disclosed below can provide eye tracking that overcomes the above limitations and can compensate for Strabismus or other personal gaze defects. The disclosed systems and methods can advantageously implement a gaze dependent eye-model that applies a different calculation process depending on a direction of the optical axis / user's gaze.

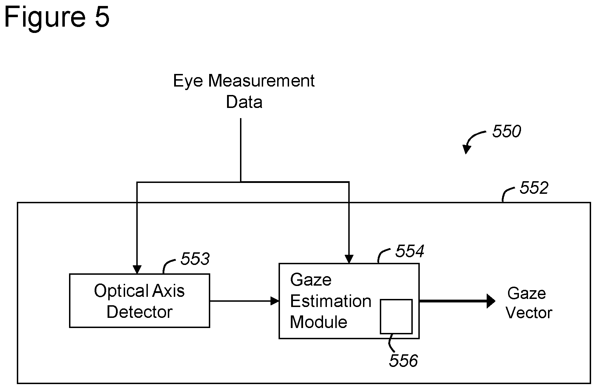

Figure 5 illustrates an eye tracking system 550 according to an embodiment of the present disclosure. The eye tracking system 550 comprises a controller 552. The controller 552 is configured to (for one or both eyes of a user) receive eye measurement data associated with an eye of a user. The eye measurement data may include images of one or both eyes, or may be derived from such images, as will be discussed below. The controller 552 comprises an optical axis detector 553 configured to receive the eye measurement data and determine an optical axis of the eye from the eye measurement data. The controller 552 also includes a gaze estimation module 554 configured to receive the optical axis from the optical axis detector 553 and select one of a plurality of eye models based on a direction of the optical axis. The gaze estimation module 554 is further configured to receive the eye measurement data and determine a gaze vector (gaze) of the eye by applying the selected eye model to the eye measurement data.

The eye measurement data may include one or more parameters determined from an image of the eye of the user. For example, the eye measurement data may comprise the position of corneal reflections, a calculated corneal centre, a determined pupil position etc, as described above. In other examples, the eye measurement data may comprise a raw image of the eye received from an image sensor. In such examples, the controller 552 is further configured to determine the corneal centre and pupil position from the raw image as described above and known in the art.

By selecting one of a plurality of eye models based on a direction of the optical axis, the eye tracking system can advantageously compensate for Strabismus for one or both eyes. In particular, the eye tracking system may implement different eye models (and therefore perform different gaze calculation processes) for Strabismus regions and regions of normal eye behaviour.

The approaches described above use a few (for example, 5) known stimulus points to calibrate a single eye-model with eye model parameters (e.g. PPO, FO). As a result, the same eye model is used for all gaze angles. The eye tracking system 550 of Figure 5 can select one of a plurality of eye models that changes its mapping of pupil centre and corneal centre to gaze vector dependent on which direction the user is looking (as determined by the detected optical axis). This can advantageously compensate for Strabismus effects in one or more directions and in one or both eyes.

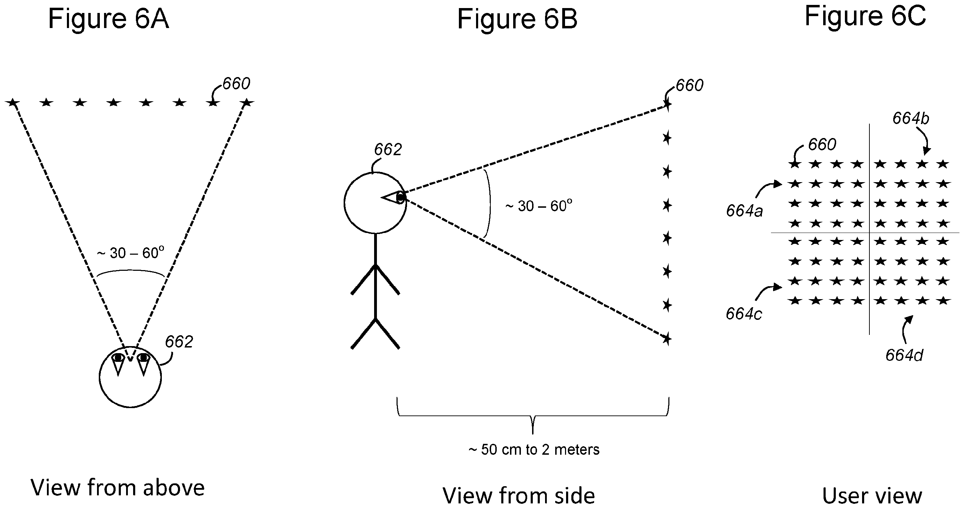

The plurality of eye models may be determined from a calibration routine, such as that illustrated in Figures 6A to 6C. The calibration routine may include presenting a series of stimulus points 660 (also referred to as calibration points) to a user 662 on a display. For a remote eye tracking system, the user may be positioned at a fixed distance from a display screen such as a computer screen. The fixed distance may range from 50 cm to 200 cm. For a head mounted eye-tracking system, the stimulus points 660 may be presented at a distance of approximately 2 meters in virtual space. In other remote or head mounted examples, the distance may be greater than 2 meters and up to infinity in virtual space. In this example, the stimulus points 660 are arranged in a 8 x 8 square grid. However, any number of stimulus points 660 can be displayed. The number of stimulus points may be greater than 5 (the number of points that can be used in a single model calibration). A high number of points is desirable for better characterisation of a user's Strabismus, however, less points is desirable to avoid a time-consuming calibration routine. The stimulus points 660 may be arranged in a 4 x 4 grid, a 4 x 5 grid, a 5 x 5 grid, a 5 x 6 grid or a 6 x 6 grid. In other examples, the stimulus points 660 may be positioned in a non-grid pattern. The size of the stimulus point pattern and the distance between the user 662 and display may be chosen such that the stimulus pattern subtends an angle of from approximately 30 to 60 degrees from the user's eyes in both the horizontal and vertical direction. The stimulus points 662 may be displayed in turn (one at a time) to the user 662. Each stimulus point 660 may be displayed for from 0.5 to 1.0 seconds. The stimulus points 660 may be displayed in any order.

In a first example, the plurality of eye models may comprise a plurality of predetermined eye models determined by the controller 552 as part of the calibration process. Such eye models can be considered as predetermined in that they are determined during a calibration process, prior to use of the eye tracking system on "live" (i.e. non-calibration) eye measurement data. As an example, during calibration, the controller 552 may determine individual eye models for each stimulus point 660 based on eye measurement data that was recorded when the user was instructed to look at each corresponding stimulus point 660. As a further example, the controller 552 may determine individual eye models for a plurality of regions of stimulus points 660. For example, the controller 552 may determine four individual eye models, one corresponding to each quadrant 664a, 664b, 664c, 664d (left upper, right upper, left lower, right lower, referred to as quadrants 664) as viewed by a user, as illustrated in Figure 6C. Each individual (predetermined) eye model (determined during the calibration process) may have its own individual model parameter sets (PPO, FO etc) calculated during calibration for each stimulus point or quadrant / region as appropriate. In this way, the controller is configured to determine a plurality of eye modelling parameter sets defining a corresponding plurality of predetermined eye models during the calibration process.

During use of the eye tracking system 550 (following calibration), the optical axis detector 553 can determine a direction of the optical axis of the eye in the usual manner, for example based on the PCCR method, to determine an approximate gaze angle of the user. In this way, the controller 552 can determine which stimulus point 660 or region of stimulus points (e.g. which quadrant 664a-664d) is associated with the determined optical axis. The gaze estimation model 554 can then select the eye model corresponding to the determined region / quadrant 664a-664d / stimulus point 660. In this way, the controller 552 can use an eye-model with different eye model parameters (PPO, FO etc) based on the determined angle of the optical axis, to account for Strabismus effects or similar eye gaze defects. If a direction of the optical axis is between two or more stimulus points 660, the controller 552 may interpolate the model parameters for the eye-models corresponding to the closest stimulus points 660. The interpolation may depend on a distance to each stimulus point 660 such that the closer stimulus point 660 is more heavily weighted in the combination.

In a second example, the plurality of eye models may comprise a baseline eye model and a plurality of gaze offset values. The controller 552 may determine the baseline eye model and the plurality of gaze offset values as part of the calibration process. The controller 552 may determine the baseline eye model from one or more stimulus points 660, for example from 5 stimulus points 660 or all stimulus points 660, using the conventional approach. Each of the plurality of gaze offset values may correspond to a respective stimulus point 660 or region of stimulus points 660. The controller 552 may determine each gaze offset value as an error value between a calculated baseline gaze angle using the baseline eye model and an expected gaze angle for the respective stimulus point or points 660. Each gaze offset value may comprise an error value in the vertical direction (y-axis) and an error value in the horizontal direction (x-axis). The plurality of gaze offset values may be referred to as an error value matrix (EVM). The size of the EVM may correspond to the size of the stimulus point grid or the number of distinct regions of stimulus points. In this way, the controller is configured to determine a plurality of gaze offset values for applying to a baseline eye model during the calibration process.

During use, following calibration, the optical axis detector 553 can determine a direction of the optical axis. The gaze estimation module 552 may then determine the stimulus point 660 (or region of stimulus points) that is closest to the determined direction of the optical axis. The gaze estimation module 552 can then select one of a plurality of eye models by selecting the gaze offset value corresponding to the stimulus point 660 closest to the direction of the optical axis, and applying the gaze offset value to the baseline model. In this way, the controller 552 can use a single eye-model with the same eye model parameters (PPO, FO etc) and apply a direction dependent gaze angle offset to account for Strabismus or similar defects. If a direction of the optical axis is between two or more stimulus points 660 (or regions of stimulus points), the controller 552 may interpolate the error vector applied to the calculated baseline gaze angle based on the gaze offset values from the closest stimulus points 660. The interpolation may depend on a distance to each stimulus point 660 or region of stimulus points 660.

The second example maintains a single value for each of the PPO and FO in the baseline model, which provides a single representation of the real physical parameters of the eye. This can advantageously avoid anomalously large numbers of PPO or FO, which can result in undesirable noise sensitivity. This approach can also advantageously provide a simpler approach than the first example by providing a single eye model with a plurality of scalar offsets.

A third example may comprise a blend of the first and second examples. In other words, the controller 552 may determine a plurality of predetermined eye models and a plurality of gaze offset values during the calibration process. Each predetermined eye model may correspond to a different region of calibration / stimulus points, for example there may be a separate eye model for each of the four quadrants 664. The controller 552 may determine each gaze offset value for each individual stimulus point in the region as an error value between a calculated gaze angle using the appropriate predetermined eye model and a known gaze angle of the respective stimulus point 660. For instance, the controller may determine an individual gaze offset value for each stimulus point in the region / quadrant, relative to the predetermined eye model for that region / quadrant. In this way, the controller is configured to determine a plurality of eye modelling parameter sets defining a corresponding plurality of predetermined eye models; and a plurality of gaze offset values for applying to one of the predetermined eye models, during the calibration process.

During subsequent use, the gaze estimation module 554 may select one of a plurality of eye models by: selecting one of a plurality of predetermined eye models based on a direction of the optical axis; selecting one of a plurality of gaze offset values based on a direction of the optical axis; and applying the selected gaze offset value to the selected predetermined eye model.

In some instances of Strabismus, eye behaviour may be inconsistent for one or more gaze angles. In some examples, the eye tracking system 550 may be configured to detect such inconsistent eye-performance such that eye tracking may be disabled for affected gaze angles for the affected eye.

It will be appreciated that the controller can perform the above calibration process, model selection and gaze vector determination for both the left eye and the right eye of the user. The controller 552 can combine the resultant eye vectors for each eye to provide a combined gaze vector. In some examples, the controller 552 may apply weightings to the left eye gaze vector and the right eye gaze vector when providing the combined gaze vector. The controller can determine the weighting for each eye based on values associated with the selected eye model for the respective eye. For example, for the second and third examples described above, the weightings may be based on a magnitude of the gaze offset value, which represents an error of the user's gaze relative to the baseline or predetermined model. The weightings may also be based on a variation of the gaze offset value relative to neighbouring gaze offset values (associated with neighbouring stimulus points). High variation in gaze offset values for a particular region (of stimulus points 660) may indicate that the eye gaze vector is erratic in that region and therefore may be an indication of Strabismus or other gaze related defects. For the first and third examples, the weightings may be based on a variation of values of the eye modelling parameter sets (e.g. PPO, FO) relative to neighbouring values of the plurality of eye modelling parameter sets (associated with neighbouring stimulus points / regions of stimulus points). The weightings may comprise a value from 0 to 1. The sum of the pair of weightings (one for each eye) may equal 1. The weightings may depend on the magnitude of the gaze offset value or the size of any variation. For regions of particularly high magnitude gaze offset values (magnitude above a threshold) or particularly high variation (variation above a threshold variation) of the gaze offset values and / or values of eye modelling parameter sets, the controller 552 may set the corresponding weightings to zero to disable eye gaze tracking in that region. In some examples, both eyes may experience the same regions of poor performance and the controller may set the corresponding weightings for both eyes to zero, disabling eye tracking altogether. In this way, the eye tracking system 550 can advantageously disable eye tracking rather than provide an inaccurate gaze value in regions of severe Strabismus. In other examples, the controller 552 may only set the weighting to zero for the eye with the highest error / variation.

In some examples, the controller 552 may determine a plurality of weightings (which may also be referred to as pairs of weighting values - one weighting value for each eye) during the calibration process. Each of the plurality of weightings may correspond to: each of the plurality of predetermined models / eye modelling parameter sets (examples 1 and 3); each of the plurality of gaze offset values (examples 2 and 3); and / or each stimulus point 660 (examples 1, 2 and 3).

The plurality of weightings may be referred to as an eye weighting matrix (EWM) with a size corresponding to the number of different eye models (which can be implemented as a plurality of different eye models and / or a plurality of different gaze offset values), which can be same as the size of the stimulus point grid.

In some examples, the controller 552 may determine the plurality of weighting values as part of a two-stage calibration process. During a first stage, a plurality of stimulus points 660 may be displayed one at a time to the user 662 and the controller 552 may determine (for each eye) a plurality of eye modelling parameter sets; and / or a plurality of gaze offset values, corresponding to each stimulus point or region of stimulus points as described above in relation to examples 1 to 3. During the second stage, the plurality of stimulus points 660 may be re-displayed one at a time to the user 662. As each stimulus point is displayed, the controller 552 may, for each eye, receive eye measurement data, select the eye model corresponding to the stimulus point and determine a calculated gaze vector using the selected eye model and eye measurement data. The controller 552 may then compare the calculated eye vector for each eye to the known eye vector corresponding to the stimulus point and determine a weighting for each eye based on a magnitude of the difference between the calculated eye vector and the known eye vector. In this way, selected eye models that do not accurately calculate the gaze vector can be given a lower weighting. This can be useful when subsequently combining gaze vectors for the left and right eyes such that gaze vectors that are expected to be less accurate have a lesser influence on the combined gaze signal. As described above, one or more weighting values for one or both eyes may be set to zero to disable gaze tracking for the relevant eye(s) during subsequent use when the user's gaze corresponds to the direction of the relevant stimulus point(s). A weighting value may be set to zero if the difference between the calculated eye vector and the known eye vector is greater than an acceptable difference threshold.

In a similar example, the first stage of the calibration process may comprise the controller 552 determining (for each eye) a first plurality of gaze offset values for a plurality of stimulus points 660 for applying to a baseline model (example 2) or one of a plurality of predetermined models (example 3). The second stage may comprise the controller 552 determining (for each eye) a second plurality of gaze offset values for the same plurality of stimulus points for applying to the same baseline model or the same one of a plurality of predetermined models. The difference between each of the first plurality of gaze offset values and the corresponding one of the second plurality of gaze offset values represents the consistency of the user's eye gaze towards that stimulus point / region of stimulus points. The controller 552 can then determine (for each eye) a plurality of weightings based on the difference between corresponding values of the gaze offset values in the first and second plurality of gaze offset values.

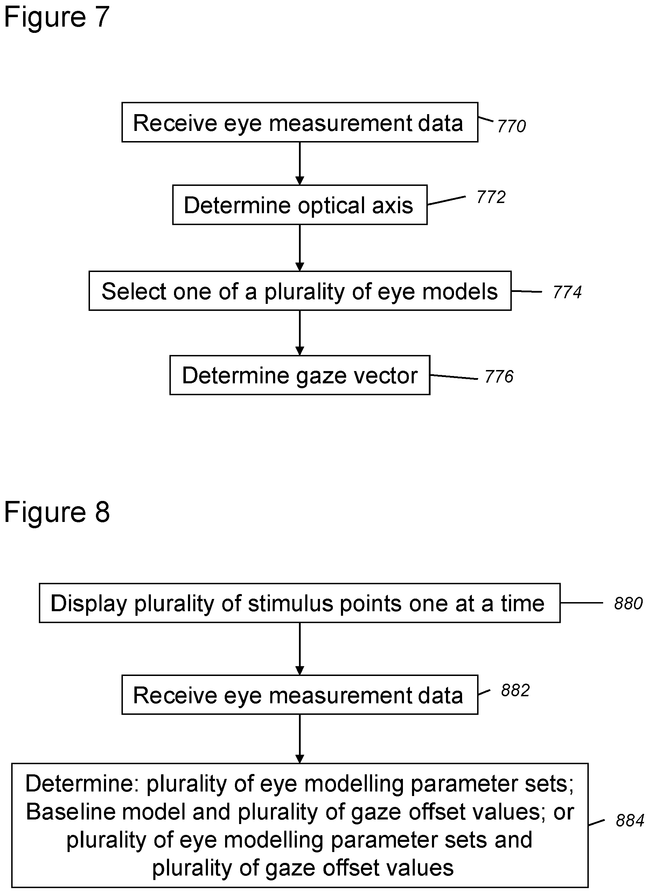

Figure 7 illustrates a method of eye tracking according to an embodiment of the present disclosure. The method can provide eye tracking that advantageously accounts for eye gaze defects such as Strabismus.

Step 770 comprises receiving eye measurement data associated with an eye of a user. Step 772 comprises determining an optical axis of the eye from the eye measurement data. Step 774 comprises selecting one of a plurality of eye models based on a direction of the optical axis. Step 776 comprises determining a gaze vector of the eye by applying the selected eye model to the eye measurement data.

Figure 8 illustrates a method of calibrating an eye tracking system according to an embodiment of the present disclosure. The method can enable an eye tracking system that can advantageously accounts for eye gaze defects such as Strabismus.

Step 880 comprises causing a plurality of stimulus points to be displayed one at a time to a user. Step 882 comprises receiving eye measurement data for each stimulus point. Step 884 comprises determining: a plurality of eye modelling parameter sets defining a corresponding plurality of predetermined eye models; a baseline eye model and a plurality of gaze offset values for applying to the baseline eye model; or a plurality of eye modelling parameter sets defining a corresponding plurality of predetermined eye models and a plurality of gaze offset values for applying to one of the predetermine eye models. Each of: the plurality of eye modelling parameter sets; and / or the plurality of gaze offset values correspond to: each stimulus point; or a region of stimulus points.

The disclosed eye tracking systems can provide a strabismus compensation mode by implementing the above-described calibration process, model definition and model selection. A user suffering from gaze defects may select the strabismus compensation mode instead of the conventional single model personal calibration.

The disclosed system and methods provide a solution for users to provide functioning eye-tracking that accounts for their individual strabismus effect.

Throughout the present specification, the descriptors relating to relative orientation and position, such as "horizontal", "vertical", "top", "bottom" and "side", are used in the sense of the orientation of the apparatus / device as presented in the drawings. However, such descriptors are not intended to be in any way limiting to an intended use of the described or claimed invention.

It will be appreciated that any reference to "close to", "before", "shortly before", "after" "shortly after", "higher than", or "lower than", etc, can refer to the parameter in question being less than or greater than a threshold value, or between two threshold values, depending upon the context.

## Claims

1. An eye tracking system (100, 550) comprising a controller (125, 552) configured to, for one or each of a left eye and a right eye of a user:
determine a plurality of eye models by determining, during a calibration process:
a plurality of eye modelling parameter sets defining a corresponding plurality of predetermined eye models;
a baseline eye model and a plurality of gaze offset values for applying to the baseline eye model; or
a plurality of eye modelling parameter sets defining a corresponding plurality of predetermined eye models and a plurality of gaze offset values for applying to one of the predetermine eye models;
receive (770) eye measurement data associated with the eye;
determine (772) an optical axis of the eye from the eye measurement data; and
select (774) one of the plurality of eye models based on a direction of the optical axis; and
determine (776) a gaze vector of the eye by applying the selected eye model to the eye measurement data.

2. The eye tracking system (100, 550) of claim 1, wherein the controller (125, 552) is configured to select one of a plurality of predetermined eye models based on the direction of the optical axis.

3. The eye tracking system (100, 550) of claim 1, wherein the controller (125, 552) is configured to select one of a plurality of eye models by:
selecting a gaze offset value from a plurality of gaze offset values based on the direction of the optical axis; and
applying the selected gaze offset value to a baseline eye model.

4. The eye tracking system of claim 2, wherein the controller is configured to select one of a plurality of eye models by:
selecting a gaze offset value from a plurality of gaze offset values based on the direction of the optical axis; and
applying the selected gaze offset value to the selected predetermined eye model.

5. The eye tracking system (100, 550) of claim 1, wherein the controller (125, 552) is configured to:
cause a plurality of stimulus points to be displayed one at a time to a user; and
receive eye measurement data for each stimulus point.

6. The eye tracking system (100, 550) of claim 5, wherein each of:
the plurality of eye modelling parameter sets; and / or
the plurality of gaze offset values,
correspond to:
each stimulus point; or
a region of stimulus points.

7. The eye tracking system (100, 550) of any preceding claim, wherein the controller (125, 552) is configured to:
determine a gaze vector for the left eye of the user;
determine a gaze vector for the right eye of the user;
determine a weighting for each of the left eye gaze vector and the right eye gaze vector based on the selected eye model for the respective left and right eye; and
apply the weighting to the gaze vector of each of the left eye and the right eye to provide a combined gaze vector.

8. The eye tracking system (100, 550) of claim 7 when dependent on any of claims 3 to 6, wherein the controller (125, 552) is configured to:
determine the weighting based on a magnitude of the gaze offset value associated with the selected eye model, and/or
determine the weighting as dependent on a variation of the gaze offset values associated with the selected eye model relative to neighbouring values of the plurality of gaze offset values, and/or
determine the weighting as dependent on a variation of the values of the eye modelling parameter sets associated with the selected eye model relative to neighbouring values of the plurality of eye modelling parameter sets.

9. The eye tracking system (100, 550) of any of claims 7 to 8, when dependent on any of claims 1 or 6, wherein the controller (125, 552) is configured to determine a plurality of weightings during the calibration process, each weighting corresponding to each of:
the plurality of eye modelling parameter sets;
the plurality of gaze offset values; and / or
the plurality of stimulus points (660).

10. The eye tracking system (100, 550) of any of claims 7 to 9, when dependent on any of claims 5 to 6, wherein the controller (125, 552) is further configured to:
cause the plurality of stimulus points to be re-displayed one at a time to the user; and
for each stimulus point that is displayed:
receive eye measurement data;
select the eye model corresponding to the stimulus point;
calculate a gaze vector using the selected eye model and the eye measurement data;
calculate a difference between the calculated gaze vector and the known gaze vector corresponding to the stimulus point; and
determine a weighting based on the difference.

11. The eye tracking system (100, 550) of claim 5, where the plurality of stimulus points (660) comprises five or more stimulus points.

12. A head-mounted device comprising the eye tracking system (100, 550) of any preceding claim.

13. A method of eye tracking, the method comprising:
determining a plurality of eye models by determining, during a calibration process:
a plurality of eye modelling parameter sets defining a corresponding plurality of predetermined eye models;
a baseline eye model and a plurality of gaze offset values for applying to the baseline eye model; or
a plurality of eye modelling parameter sets defining a corresponding plurality of predetermined eye models and a plurality of gaze offset values for applying to one of the predetermine eye models;
receiving (770) eye measurement data associated with an eye of a user (662);
determining (772) an optical axis of the eye from the eye measurement data; and
selecting (774) one of the plurality of eye models based on a direction of the optical axis; and
determining (776) a gaze vector of the eye by applying the selected eye model to the eye measurement data.

14. A method of calibrating an eye tracking system, the method comprising:
causing (880) a plurality of stimulus points to be displayed in different gaze directions one at a time to a user;
receiving (882) eye measurement data for one eye, for each stimulus point (660);
determining (884):
a plurality of eye modelling parameter sets defining a corresponding plurality of predetermined eye models;
a baseline eye model and a plurality of gaze offset values for applying to the baseline eye model; or
a plurality of eye modelling parameter sets defining a corresponding plurality of predetermined eye models and a plurality of gaze offset values for applying to one of the predetermine eye models,
wherein each of:
the plurality of eye modelling parameter sets; and / or
the plurality of gaze offset values,
correspond to:
each stimulus point (660); or
a region of stimulus points.

15. One or more non-transitory computer-readable storage media storing computer-executable instructions that, when executed by a computing system, causes the computing system to perform the method of claim 13 or claim 14.

## Patentansprüche

1. Augenverfolgungsystem (100, 550), umfassend eine Steuerung (125, 552), die konfiguriert ist, für eines oder jedes eines linken Auges und eines rechten Auges eines Benutzers:
Bestimmen einer Vielzahl von Augenmodellen durch Bestimmen, während eines Kalibrierungsprozesses:
einer Vielzahl von Augenmodellierungsparametersätzen, die eine entsprechende Vielzahl von zuvor bestimmten Augenmodellen definiert;
eines Basislinienaugenmodells und einer Vielzahl von Blickversatzwerten zum Anwenden auf das Basislinienaugenmodell; oder
einer Vielzahl von Augenmodellierungsparametersätzen, die eine entsprechende Vielzahl von zuvor bestimmten Augenmodellen und eine Vielzahl von Blickversatzwerten zum Anwenden auf eines der zuvor bestimmten Augenmodelle definiert;
Empfangen (770) von Augenmessdaten, die mit dem Auge verknüpft sind;
Bestimmen (772) einer optischen Achse des Auges aus den Augenmessdaten; und
Auswählen (774) eines der Vielzahl von Augenmodellen basierend auf einer Richtung der optischen Achse; und
Bestimmen (776) eines Blickvektors des Auges durch Anwenden des ausgewählten Augenmodells auf die Augenmessdaten.

2. Augenverfolgungssystem (100, 550) nach Anspruch 1, wobei die Steuerung (125, 552) konfiguriert ist, um basierend auf der Richtung der optischen Achse eines von einer Vielzahl von zuvor bestimmten Augenmodellen auszuwählen.

3. Augenverfolgungssystem (100, 550) nach Anspruch 1, wobei die Steuerung (125, 552) konfiguriert ist, um eines von einer Vielzahl von Augenmodellen auszuwählen durch:
Auswählen eines Blickversatzwerts aus einer Vielzahl von Blickversatzwerten basierend auf der Richtung der optischen Achse; und
Anwenden des ausgewählten Blickversatzwerts auf ein Basisaugenmodell.

4. Augenverfolgungssystem nach Anspruch 2, wobei die Steuerung konfiguriert ist, um eines von einer Vielzahl von Augenmodellen auszuwählen durch:
Auswählen eines Blickversatzwerts aus einer Vielzahl von Blickversatzwerten basierend auf der Richtung der optischen Achse; und
Anwenden des ausgewählten Blickversatzwerts auf das ausgewählte zuvor bestimmte Augenmodell.

5. Augenverfolgungssystem (100, 550) nach Anspruch 1, wobei die Steuerung (125, 552) konfiguriert ist zum:
Veranlassen, dass einem Benutzer eine Vielzahl von Reizpunkten einer nach dem anderen angezeigt werden; und
Empfangen von Augenmessdaten für jeden Reizpunkt.

6. Augenverfolgungssystem (100, 550) nach Anspruch 5, wobei jedes von:
der Vielzahl von Augenmodellierungsparametersätzen; und/oder
der Vielzahl von Blickversatzwerten,
entsprechen:
jedem Reizpunkt; oder
einer Region von Reizpunkten.

7. Augenverfolgungssystem (100, 550) nach einem der vorstehenden Ansprüche, wobei die Steuerung (125, 552) konfiguriert ist zum:
Bestimmen eines Blickvektors für das linke Auge des Benutzers;
Bestimmen eines Blickvektors für das rechte Auge des Benutzers;
Bestimmen einer Gewichtung für jeden der Blickvektoren des linken Auges und des rechten Auges basierend auf dem ausgewählten Augenmodell für das jeweilige linke und rechte Auge; und
Anwenden der Gewichtung auf den Blickvektor jedes des linken und des rechten Auges, um einen kombinierten Blickvektor bereitzustellen.

8. Augenverfolgungssystem (100, 550) nach Anspruch 7, in Abhängigkeit von einem der Ansprüche 3 bis 6, wobei die Steuerung (125, 552) konfiguriert ist zum:
Bestimmen der Gewichtung basierend auf einer Größe des Blickversatzwerts, der mit dem ausgewählten Augenmodell verknüpft ist, und/oder
Bestimmen der Gewichtung in Abhängigkeit von einer Variation der Blickversatzwerte, die mit dem ausgewählten Augenmodell verknüpft sind, relativ zu benachbarten Werten der Vielzahl von Blickversatzwerten, und/oder
Bestimmen der Gewichtung in Abhängigkeit von einer Variation der Werte der Augenmodellierungsparametersätze, die mit dem ausgewählten Augenmodell verknüpft sind, relativ zu benachbarten Werten der Vielzahl von Augenmodellierungsparametersätzen.

9. Augenverfolgungssystem (100, 550) nach einem der Ansprüche 7 bis 8, in Abhängigkeit von einem der Ansprüche 1 oder 6, wobei die Steuerung (125, 552) konfiguriert ist, um während des Kalibrierungsprozesses eine Vielzahl von Gewichtungen zu bestimmen, wobei jede Gewichtung jedem entspricht von:
der Vielzahl von Augenmodellierungsparametersätzen;
der Vielzahl von Blickversatzwerten; und / oder
der Vielzahl von Reizpunkten (660).

10. Augenverfolgungssystem (100, 550) nach einem der Ansprüche 7 bis 9, in Abhängigkeit von einem der Ansprüche 5 bis 6, wobei die Steuerung (125, 552) ferner konfiguriert ist zum:
Veranlassen, dass dem Benutzer die Vielzahl der Stimulus-Punkte einer nach dem anderen erneut angezeigt wird; und
für jeden Reizpunkt, der angezeigt wird:
Empfangen von Augenmessdaten;
Auswählen des Augenmodells, das dem Reizpunkt entspricht;
Berechnen eines Blickvektors unter Verwendung des ausgewählten Augenmodells und der Augenmessdaten;
Berechnen eines Unterschieds zwischen dem berechneten Blickvektor und dem bekannten Blickvektor, der dem Reizpunkt entspricht; und
Bestimmen einer Gewichtung basierend auf dem Unterschied.

11. Augenverfolgungssystem (100, 550) nach Anspruch 5, wobei die Vielzahl von Reizpunkten (660) fünf oder mehr Reizpunkte umfasst.

12. Kopfmontierte Vorrichtung, umfassend das Augenverfolgungssystem (100, 550) nach einem der vorstehenden Ansprüche.

13. Verfahren für eine Augenverfolgung, das Verfahren umfassend:
Bestimmen einer Vielzahl von Augenmodellen durch Bestimmen, während eines Kalibrierungsprozesses:
einer Vielzahl von Augenmodellierungsparametersätzen, die eine entsprechende Vielzahl von zuvor bestimmten Augenmodellen definiert;
eines Basislinienaugenmodells und einer Vielzahl von Blickversatzwerten zum Anwenden auf das Basislinienaugenmodell; oder
einer Vielzahl von Augenmodellierungsparametersätzen, die eine entsprechende Vielzahl von zuvor bestimmten Augenmodellen und eine Vielzahl von Blickversatzwerten zum Anwenden auf eines der zuvor bestimmten Augenmodelle definiert;
Empfangen (770) von Augenmessdaten, die mit einem Auge eines Benutzers (662) verknüpft sind;
Bestimmen (772) einer optischen Achse des Auges aus den Augenmessdaten; und
Auswählen (774) eines der Vielzahl von Augenmodellen basierend auf einer Richtung der optischen Achse; und
Bestimmen (776) eines Blickvektors des Auges durch Anwenden des ausgewählten Augenmodells auf die Augenmessdaten.

14. Verfahren zum Kalibrieren eines Augenverfolgungssystems, das Verfahren umfassend:
Veranlassen (880), dass einem Benutzer eine Vielzahl von Reizpunkten einer nach dem anderen in unterschiedlichen Blickrichtungen angezeigt werden;
Empfangen (882) von Augenmessdaten für ein Auge für jeden Reizpunkt (660);
Bestimmen (884):
einer Vielzahl von Augenmodellierungsparametersätzen, die eine entsprechende Vielzahl von zuvor bestimmten Augenmodellen definiert;
eines Basislinienaugenmodells und einer Vielzahl von Blickversatzwerten zum Anwenden auf das Basislinienaugenmodell; oder
einer Vielzahl von Augenmodellierungsparametersätzen, die eine entsprechende Vielzahl von zuvor bestimmten Augenmodellen und eine Vielzahl von Blickversatzwerten zum Anwenden auf eines der zuvor bestimmten Augenmodelle definiert,
wobei jedes von:
der Vielzahl von Augenmodellierungsparametersätzen; und / oder
der Vielzahl von Blickversatzwerten,
entsprechen:
jedem Reizpunkt (660); oder
einer Region von Reizpunkten.

15. Ein oder mehrere nicht-flüchtige computerlesbare Speichermedien, die computerausführbare Anweisungen speichern, die, wenn sie durch ein Rechensystem ausgeführt werden, das Rechensystem veranlassen, das Verfahren nach Anspruch 13 oder 14 durchzuführen.

## Revendications

1. Système de suivi oculaire (100, 550) comprenant un contrôleur (125, 552) configuré, pour l'un ou chacun parmi un oeil gauche et un oeil droit d'un utilisateur, pour :
déterminer une pluralité de modèles d'oeil en déterminant, pendant un processus d'étalonnage :
une pluralité d'ensembles de paramètres de modélisation d'oeil définissant une pluralité correspondante de modèles d'oeil prédéterminés ;
un modèle d'oeil de ligne de base et une pluralité de valeurs de décalage de regard pour application au modèle d'oeil de ligne de base ; ou
une pluralité d'ensembles de paramètres de modélisation d'oeil définissant une pluralité correspondante de modèles d'oeil prédéterminés et une pluralité de valeurs de décalage de regard pour application à l'un des modèles d'oeil prédéterminés ;
recevoir (770) des données de mesure d'oeil associées à l'oeil ;
déterminer (772) un axe optique de l'oeil à partir des données de mesure d'oeil ; et
sélectionner (774) l'un parmi la pluralité de modèles d'oeil en fonction d'une direction de l'axe optique ; et
déterminer (776) un vecteur de regard de l'oeil en appliquant le modèle d'oeil sélectionné aux données de mesure d'oeil.

2. Système de suivi oculaire (100, 550) selon la revendication 1, dans lequel le contrôleur (125, 552) est configuré pour sélectionner l'un parmi une pluralité de modèles d'oeil prédéterminés en fonction de la direction de l'axe optique.

3. Système de suivi oculaire (100, 550) selon la revendication 1, dans lequel le contrôleur (125, 552) est configuré pour sélectionner l'un parmi une pluralité de modèles d'oeil par :
la sélection d'une valeur de décalage de regard parmi une pluralité de valeurs de décalage de regard en fonction de la direction de l'axe optique ; et
l'application de la valeur de décalage de regard sélectionnée à un modèle d'oeil de ligne de base.

4. Système de suivi oculaire selon la revendication 2, dans lequel le contrôleur est configuré pour sélectionner l'un parmi une pluralité de modèles d'oeil par :
la sélection d'une valeur de décalage de regard parmi une pluralité de valeurs de décalage de regard en fonction de la direction de l'axe optique ; et
l'application de la valeur de décalage de regard sélectionnée au modèle d'oeil prédéterminé sélectionné.

5. Système de suivi oculaire (100, 550) selon la revendication 1, dans lequel le contrôleur (125, 552) est configuré pour :
amener une pluralité de points de stimulation à être affichés un à la fois à un utilisateur ; et
recevoir des données de mesure d'oeil pour chaque point de stimulation.

6. Système de suivi oculaire (100, 550) selon la revendication 5, dans lequel chacun parmi :
la pluralité d'ensembles de paramètres de modélisation d'oeil ; et / ou
la pluralité de valeurs de décalage de regard,
correspond à :
chaque point de stimulation ; ou à
une région de points de stimulation.

7. Système de suivi oculaire (100, 550) selon l'une quelconque revendication précédente, dans lequel le contrôleur (125, 552) est configuré pour :
déterminer un vecteur de regard pour l'oeil gauche de l'utilisateur ;
déterminer un vecteur de regard pour l'oeil droit de l'utilisateur ;
déterminer une pondération pour chacun du vecteur de regard d'oeil gauche et du vecteur de regard d'oeil droit en fonction du modèle d'oeil sélectionné pour l'oeil gauche et droit respectif ; et
appliquer la pondération au vecteur de regard de chacun de l'oeil gauche et de l'oeil droit pour fournir un vecteur de regard combiné.

8. Système de suivi oculaire (100, 550) selon la revendication 7 prise en dépendance de l'une quelconque des revendications 3 à 6, dans lequel le contrôleur (125, 552) est configuré pour :
déterminer la pondération en fonction d'une grandeur de la valeur de décalage de regard associée au modèle d'oeil sélectionné, et/ou
déterminer la pondération comme dépendant d'une variation des valeurs de décalage de regard associées au modèle d'oeil sélectionné par rapport à des valeurs voisines de la pluralité de valeurs de décalage de regard, et/ou
déterminer la pondération comme dépendant d'une variation des valeurs des ensembles de paramètres de modélisation d'oeil associés au modèle d'oeil sélectionné par rapport à des valeurs voisines de la pluralité d'ensembles de paramètres de modélisation d'oeil.

9. Système de suivi oculaire (100, 550) selon l'une quelconque des revendications 7 à 8, prise en dépendance de l'une quelconque des revendications 1 ou 6, dans lequel le contrôleur (125, 552) est configuré pour déterminer une pluralité de pondérations pendant le processus d'étalonnage, chaque pondération correspondant à chacun parmi :
la pluralité d'ensembles de paramètres de modélisation d'oeil ;
la pluralité de valeurs de décalage de regard ; et / ou
la pluralité de points de stimulation (660).

10. Système de suivi oculaire (100, 550) selon l'une quelconque des revendications 7 à 9, prise en dépendance de l'une quelconque des revendications 5 à 6, dans lequel le contrôleur (125, 552) est configuré en outre pour :
amener la pluralité de points de stimulation à être réaffichés un à la fois à l'utilisateur ; et
pour chaque point de stimulation qui est affiché :
recevoir des données de mesure d'oeil ;
sélectionner le modèle d'oeil correspondant au point de stimulation ;
calculer un vecteur de regard à l'aide du modèle d'oeil sélectionné et des données de mesure d'oeil ;
calculer une différence entre le vecteur de regard calculé et le vecteur de regard connu correspondant au point de stimulation ; et
déterminer une pondération en fonction de la différence.

11. Système de suivi oculaire (100, 550) selon la revendication 5, où la pluralité de points de stimulation (660) comprend cinq points de stimulation ou plus.

12. Dispositif monté sur la tête comprenant le système de suivi oculaire (100, 550) selon l'une quelconque revendication précédente.

13. Procédé de suivi oculaire, le procédé comprenant :
la détermination d'une pluralité de modèles d'oeil en déterminant, pendant un processus d'étalonnage :
une pluralité d'ensembles de paramètres de modélisation d'oeil définissant une pluralité correspondante de modèles d'oeil prédéterminés ;
un modèle d'oeil de ligne de base et une pluralité de valeurs de décalage de regard pour application au modèle d'oeil de ligne de base ; ou
une pluralité d'ensembles de paramètres de modélisation d'oeil définissant une pluralité correspondante de modèles d'oeil prédéterminés et une pluralité de valeurs de décalage de regard pour application à l'un des modèles d'oeil prédéterminés ;
la réception (770) de données de mesure d'oeil associées à un oeil d'un utilisateur (662) ;
la détermination (772) d'un axe optique de l'oeil à partir des données de mesure d'oeil ; et
la sélection (774) de l'un parmi la pluralité de modèles d'oeil en fonction d'une direction de l'axe optique ; et
la détermination (776) d'un vecteur de regard de l'oeil en appliquant le modèle d'oeil sélectionné aux données de mesure d'oeil.

14. Procédé d'étalonnage d'un système de suivi oculaire, le procédé comprenant :
le fait d'amener (880) une pluralité de points de stimulation à être affichés dans différentes directions de regard un à la fois à un utilisateur ;
la réception (882) de données de mesure d'oeil pour un oeil, pour chaque point de stimulation (660) ;
le fait de déterminer (884) :
une pluralité d'ensembles de paramètres de modélisation d'oeil définissant une pluralité correspondante de modèles d'oeil prédéterminés ;
un modèle d'oeil de ligne de base et une pluralité de valeurs de décalage de regard pour application au modèle d'oeil de ligne de base ; ou
une pluralité d'ensembles de paramètres de modélisation d'oeil définissant une pluralité correspondante de modèles d'oeil prédéterminés et une pluralité de valeurs de décalage de regard pour application à l'un des modèles d'oeil prédéterm inés,
dans lequel chacun parmi :
la pluralité d'ensembles de paramètres de modélisation d'oeil ; et / ou
la pluralité de valeurs de décalage de regard,
correspond à :
chaque point de stimulation (660) ; ou
une région de points de stimulation.

15. Support(s) de stockage non transitoire lisible(s) par ordinateur stockant des instructions exécutables par ordinateur qui, lors d'une exécution par un système informatique, amène(nt) le système informatique à mettre en oeuvre le procédé selon la revendication 13 ou la revendication 14.
